# EUROPÄISCHE PATENTSCHRIFT

(11) **EP 3 863 569 B1**
(45) Veröffentlichungstag und Bekanntmachung des Hinweises auf die Patenterteilung: **19.04.2023**
(21) Anmeldenummer: 19786747.6
(22) Anmeldetag: 08.10.2019
(51) Int. Cl.: A61F 2/46

(54) **EINSCHLAG-INSTRUMENT**
TAP-IN INSTRUMENT
INSTRUMENT À PERCUSSION

(30) Priorität: 11.10.2018 DE 102018125190
(43) Veröffentlichungstag der Anmeldung: 18.08.2021
(73) Patentinhaber: Endocon GmbH, 69257 Wiesenbach (DE); Universitätsklinikum Heidelberg, 69120 Heidelberg (DE)
(72) Erfinder: MÜLLER, Ulrike, 15526 Bad Saarow (DE); KRETZER, Jan Philippe, 69120 Heidelberg (DE); NOTARBARTOLO, Klaus, 69257 Wiesenbach (DE); LINGSLEBE, Robert, 69118 Heidelberg (DE)
(74) Vertreter: Reiser & Partner Patentanwälte mbB
(86) Internationale Anmeldenummer: PCT/EP2019/077203
(87) Internationale Veröffentlichungsnummer: WO 2020/074501

(56) Entgegenhaltungen:
- DE-A1-102016 101 900
- DE-U1-202006 000 845
- US-A1- 2015 094 728

## Beschreibung

Die Erfindung betrifft ein Einschlag-Instrument zum Fügen prothetischer Implantate, umfassend ein Gehäuse, welches einen Schlagbolzen aufnimmt, wobei dem Schlagbolzen ein erstes Federelement zugeordnet ist, wobei der Schlagbolzen mittels einer Rastvorrichtung in dem Gehäuse fixiert ist, wobei das erste Federelement den Schlagbolzen nach Lösen der Rastvorrichtung in Richtung auf ein Schlagstück beschleunigt und einen Impuls in das Schlagstück einleitet.

Ein derartiges Einschlag-Instrument ist aus der DE 20 2006 000 845 U1 bekannt. Das Einschlag-Instrument dient vorwiegend dazu, ein Implantatteil, beispielsweise eine Gelenkkugel, auf einen konischen Klemmzapfen eines anderen Implantatteiles zu befestigen. Das aus dem Stand der Technik vorbekannte Einschlag-Instrument ermöglicht das Ausüben eines vorbestimmten Impulses, welcher nahezu ausschließlich von der Federkraft der in dem Einschlag-Instrument integrierten Feder abhängt.

Ein Problem besteht jedoch darin, dass das vorbekannte Einschlag-Instrument zunächst manuell vorgespannt werden muss. Das Auslösen erfolgt durch Drücken eines Auslöseknopfes. Danach wird zwar ein Schlagbolzen beschleunigt und übt einen vorbestimmten Impuls auf ein Schlagstück aus, dies erfolgt aber unabhängig von der Positionierung des Schlagstückes auf das zu befestigende Implantatteil. Dadurch ist nicht sicher gewährleistet, dass der von dem Schlagbolzen auf das Schlagstück ausgeübte Impuls durch das Schlagstück vollständig auf das zu fügende Implantatteil übertragen wird.

Der Erfindung liegt die Aufgabe zugrunde, ein Einschlag-Instrument bereitzustellen, welches bei einfacher Handhabung einen reproduzierbaren Impulseintrag auf ein zu fügendes Implantatteil ermöglicht.

Diese Aufgabe wird mit den Merkmalen von Anspruch 1 gelöst. Auf vorteilhafte Ausgestaltungen nehmen die Unteransprüche Bezug.

Das erfindungsgemäße Einschlag-Instrument zum Fügen prothetischer Implantate umfasst ein Gehäuse, welches einen Schlagbolzen, ein dem Schlagbolzen zugeordnetes erstes Federelement, eine Auslösevorrichtung und zumindest teilweise ein Schlagstück aufnimmt, wobei das Schlagstück an einer Stirnseite aus dem Gehäuse hervorsteht, wobei die Auslösevorrichtung zwischen einer Fixierposition und einer Freigabeposition beweglich ist, wobei der Schlagbolzen in der Fixierposition von dem Schlagstück beabstandet ist, wobei sich die Auslösevorrichtung bei einer axialen Bewegung des Schlagstücks in das Gehäuse hinein selbsttätig von der Fixierposition in die Freigabeposition bewegt, um nach Erreichen der Freigabeposition den Schlagbolzen freizugeben, so dass sich dieser von dem ersten Federelement beschleunigt in Richtung auf das Schlagstück bewegt.

Dementsprechend erfolgt das Auslösen des Schlagbolzens selbsttätig in Abhängigkeit der Position des Schlagstücks relativ zu dem Gehäuse. Insbesondere ist es bei dem erfindungsgemäßen Einschlag-Instrument nicht erforderlich, dass manuell ein Auslösehebel, ein Druckknopf oder dergleichen betätigt wird. Zum Fügen prothetischer Implantate setzt der Benutzer das Schlagstück auf das zu fügende prothetische Implantat auf und bewegt das Gehäuse in axialer Richtung in Richtung auf das prothetische Implantat. Dabei bewegt sich das Schlagstück in das Gehäuse hinein, wobei die Auslösevorrichtung von der Fixierposition in die Freigabeposition bewegt wird, wobei nach Erreichen der Freigabeposition der Schlagbolzen freigegeben wird und sich beschleunigt durch das erste Federelement in Richtung auf das Schlagstück bewegt, um dort einen Impuls in das Schlagstück einzubringen. Dadurch, dass die Auslösevorrichtung nur dann in die Freigabeposition bewegt wird, wenn sich das Schlagstück in das Gehäuse hineinbewegt, ist gewährleistet, dass das Einschlag-Instrument, beziehungsweise das Schlagstück, fest und sicher auf das prothetische Implantat aufgesetzt ist. Hierbei ist vorteilhaft, dass der von dem Schlagbolzen in das Schlagstück eingebrachte Impuls vollständig von dem Schlagstück auf das prothetische Implantat übertragen wird. Die Kraft, die dabei während des Bewegens des Einschlag-Instrumentes, beziehungsweise der Auslösevorrichtung, zwischen der Fixierposition und der Freigabeposition auf das prothetische Implantat einwirkt, ist dabei stets gleich. Dadurch ist es möglich, einen reproduzierbaren Impuls in das prothetische Implantat einzubringen, wobei der Impuls genau dem zum Fügen erforderlichen Impuls entspricht.

Das erfindungsgemäße Einschlag-Instrument eignet sich somit insbesondere zum Fügen von Kegelverbindungen beziehungsweise von Kegelpressverbänden, welche insbesondere im Bereich der Endoprothetik, beispielsweise bei Hüftprothesen, häufig Verwendung finden. Dort wird eine keramische oder metallische Kugel auf einen Konus eines Hüftimplantats aufgesetzt. Bislang wird die Kugel zumeist mittels eines einzigen Hammerschlages auf dem Implantat befestigt.

Bei Befestigen des Elementes mittels eines Hammerschlages ist insbesondere problematisch, dass die Kraft des Hammerschlages nicht reproduzierbar ist. Ist der ausgeübte Impuls nicht ausreichend groß, kann es vorkommen, dass die Kugel nicht ausreichend auf dem Hüftimplantat befestigt ist. Dies kann zu Mikrobewegungen zwischen Kugel und Schaft führen, wobei Metallionen freigesetzt werden können, welche das umliegende Gewebe schädigen können. Ist der ausgeübte Impuls wiederum zu groß, kann es zu Schäden am Knochen kommen, in welchen der Schaft eingesetzt ist.

Vor diesem Hintergrund ist das erfindungsgemäße Einschlag-Instrument besonders vorteilhaft, weil zum Auslösen des Einschlag-Instrumentes eine Vorspannung des Schlagstücks auf das zu befestigende Element gegeben ist. Nach Erreichen der Freigabeposition wird der Schlagbolzen schlagartig freigegeben und es wird ein Impuls mit einer sehr kurzen Impulsdauer in die Komponenten des Implantates eingebracht, um die Verbindung zu fügen.

Die Auslösevorrichtung kann eine erste Hülse aufweisen, welche axial beweglich in dem Gehäuse angeordnet ist und den Schlagbolzen aufnimmt, wobei die erste Hülse am Umfang zumindest eine Durchbrechung aufweist, wobei in der Durchbrechung zumindest ein Sperrkörper angeordnet ist. Bei dieser Ausgestaltung ist die Auslösevorrichtung in dem Gehäuse aufgenommen und unmittelbar dem Schlagbolzen zugeordnet.

In den Schlagbolzen kann zumindest eine Vertiefung eingebracht sein, welche in der Fixierposition den Sperrkörper aufnimmt. Der in die Vertiefung aufgenommene Sperrkörper bewirkt, dass der Schlagbolzen in der Fixierposition relativ zu der ersten Hülse fixiert ist.

Die Vertiefung kann in Form einer umlaufenden Nut ausgebildet sein, welche in den Umfang des Schlagbolzens eingebracht ist. Eine umlaufende Nut ist einerseits einfach herstellbar und lässt andererseits Rotationsbewegungen des Schlagbolzens zu. Dadurch kann verhindert werden, dass sich der Schlagbolzen in der ersten Hülse verklemmt.

Der Sperrkörper kann sich in der Fixierposition an die Innenwand des Gehäuses anlegen, wobei der Innendurchmesser des Gehäuses so gewählt ist, dass der Sperrkörper in der Durchbrechung der ersten Hülse und in der Vertiefung des Schlagbolzens gehalten ist und dadurch der Schlagbolzen relativ zu der ersten Hülse verriegelt ist. Das Gehäuse weist eine Durchgangsbohrung auf, wobei der Innendurchmesser der Durchgangsbohrung bei dieser Ausgestaltung so gewählt ist, dass die erste Hülse mit ihrer Außenwand an der Innenwand der Bohrung anliegt. Dadurch sind die Sperrkörper in der Durchbrechung und in der Vertiefung des Schlagbolzens fixiert, so dass der Schlagbolzen relativ zur ersten Hülse verriegelt ist.

Die Sperrkörper sind vorzugsweise in Form von Kugeln ausgebildet, wobei die erste Hülse mehrere Durchbrechungen, vorzugsweise drei über den Umfang verteilte Durchbrechungen in Form von Bohrungen, aufweist, welche die kugelförmigen Sperrkörper aufnehmen. Kugelförmige Sperrkörper sind besonders vorteilhaft, da diese an der Innenwand des Gehäuses abrollen können und nicht zum Verklemmen neigen.

Das Schlagstück kann die erste Hülse und den mit der ersten Hülse verriegelten Schlagbolzen in dem Gehäuse axial verschieben, wenn sich das Schlagstück in axialer Richtung in das Gehäuse hinein verschiebt. Beim Aufsetzen des Einschlag-Instrumentes auf das zu fügende Implantat wird eine Kraft in das Einschlag-Instrument eingebracht, wodurch sich das Schlagstück in das Gehäuse hinein verschiebt. Gleichzeitig verschieben sich damit die erste Hülse und der mit der ersten Hülse verriegelte Schlagbolzen.

Das Gehäuse kann mit einer Querschnittserweiterung versehen sein, wobei sich der Innendurchmesser des Gehäuses im Bereich der Querschnittserweiterung vergrößert, wobei die Querschnittserweiterung zur teilweisen Aufnahme des Sperrkörpers in der Freigabeposition ausgebildet ist.

Die Querschnittserweiterung ist vorzugsweise in Form einer kegelförmigen Stufe ausgebildet, welche in die Innenwand des Gehäuses eingebracht ist. Wird das Einschlag-Instrument so weit auf das Implantat aufgedrückt, dass der in der ersten Hülse fixierte Sperrkörper den Bereich der Querschnittserweiterung erreicht, gleitet der Sperrkörper aus der Vertiefung des Schlagbolzens heraus und in die Querschnittserweiterung hinein. Dadurch wird der Schlagbolzen freigegeben. Insofern entspricht der Bereich der Querschnittserweiterung der Freigabeposition.

Die erste Hülse kann auf einer Stirnseite eine Aufnahme für das Schlagstück aufweisen, wobei die Aufnahme so ausgebildet ist, dass der Schlagbolzen in der Freigabeposition das Schlagstück unmittelbar kontaktiert. Dadurch ist sichergestellt, dass der Impuls des beschleunigten Schlagbolzens vollständig in das Schlagstück übertragen werden kann.

Der Schlagbolzen kann mit einem umlaufenden Kragen versehen sein, wobei sich das erste Federelement an dem Kragen und an dem Gehäuse abstützt. Bei dieser Ausgestaltung ist das erste Federelement sicher auf dem Schlagbolzen fixiert.

Zwischen der ersten Hülse und dem Gehäuse kann eine zweite Hülse angeordnet sein, wobei die Innenwand der zweiten Hülse eine Gleitfläche für den Sperrkörper bildet und wobei die Querschnittserweiterung in die zweite Hülse eingebracht ist. Bei dieser Ausgestaltung gleitet der Sperrkörper an der Innenwand der zweiten Hülse entlang. Der Sperrkörper kontaktiert dabei insbesondere nicht die Innenwand des Gehäuses. Die Fixierposition und die Freigabeposition sind dabei durch die Ausgestaltung der Innenwand der zweiten Hülse und die Anordnung der Sperrkörper relativ zur zweiten Hülse repräsentiert.

Die zweite Hülse kann axial verschieblich in dem Gehäuse aufgenommen sein und ein zweites Federelement kann vorgesehen sein, welches die zweite Hülse relativ zu dem Gehäuse positioniert. Das zweite Federelement bewirkt, dass sich die zweite Hülse so weit bewegt, dass die Durchbrechung, die Sperrkörper und die Vertiefung fluchten und dadurch die Sperrkörper wieder in die Vertiefung hineingleiten und so den Schlagbolzen mit der ersten Hülse verriegeln.

Es kann ein drittes Federelement vorgesehen sein, welches stirnseitig an der ersten Hülse und an dem Gehäuse angeordnet ist und in der Fixierposition den Schlagbolzen und das Schlagstück voneinander beabstandet. Das dritte Federelement stellt sicher, dass die erste Hülse und das Schlagstück in die Freigabeposition zurückgeführt werden.

Das Gehäuse kann mit einem Handstück versehen sein. Das Handstück verbessert die Bedienbarkeit des Einschlag-Instrumentes.

Zwischen Gehäuse und Schlagstück kann zumindest ein Dichtelement angeordnet sein.

Gemäß einer alternativen Ausgestaltung ist in dem Gehäuse eine Hülse drehbar angeordnet, wobei die Hülse zumindest eine L-förmige Durchbrechung und eine schräge Ebene aufweist. In die Durchbrechung greift ein Mitnehmer ein, welcher beim axialen Verschieben des Gehäuses relativ zu dem Schlagstück an der schiefen Ebene entlanggleitet und dadurch ein Verdrehen der Hülse bewirkt. In der Hülse ist ferner ein Haltestift angeordnet, welcher dem Schlagbolzen zugeordnet ist. Der Haltestift gleitet bei der Axialbewegung des Gehäuses zunächst an dem horizontalen Abschnitt der L-förmigen Durchbrechung entlang und wird nach Erreichen der Kante, dem Übergang zwischen horizontalem und vertikalem Abschnitt der Durchbrechung, freigegeben. Dabei wird der Schlagbolzen durch das dem Schlagbolzen zugeordnete Federelement in Richtung auf das Schlagstück beschleunigt und ein Impuls wird auf das zu fügende Implantat ausgeübt. Eine Rückstellfeder bringt die Komponenten nach dem Entlasten selbsttätig in die Ausgangsposition zurück.

Einige Ausgestaltungen des erfindungsgemäßen Einschlag-Instrumentes werden nachfolgend anhand der Figuren näher erläutert. Die Figuren zeigen, jeweils schematisch:
Fig. 1 im Schnitt das Einschlag-Instrument in der Fixierposition;
Fig. 2 im Schnitt das Einschlag-Instrument in der Freigabeposition.

Die Figuren zeigen ein Einschlag-Instrument 1 zum Fügen prothetischer Implantate. Das Einschlag-Instrument 1 dient insbesondere dem Fügen von Kegelpressverbänden, welche allgemein in der Endoprothetik häufig Anwendung finden. Bei der vorliegenden Ausgestaltung dient das Einschlag-Instrument 1 zum Fügen von Gelenkkugeln auf einen konischen Klemmzapfen eines Hüftimplantats. Es ist aber auch denkbar, dass das Einschlag-Instrument 1 zum Fügen von Knieprothesen, Schulterprothesen oder dergleichen zum Einsatz gelangt.

Das Einschlag-Instrument 1 umfasst ein Gehäuse 2 aus metallischem Werkstoff, wobei das Gehäuse 2 mit einer Durchgangsbohrung 22 versehen ist. Das Gehäuse 2 nimmt einen Schlagbolzen 3, ein dem Schlagbolzen 3 zugeordnetes erstes Federelement 4, eine Auslösevorrichtung 5 und teilweise ein Schlagstück 6 auf. Das Schlagstück 6 ragt an einer Stirnseite des Gehäuses 2 aus dem Gehäuse 2 heraus.

Der Schlagbolzen 3, das erste Federelement 4 und das Schlagstück 6 sind ebenfalls aus metallischem Werkstoff ausgebildet.

Die Auslösevorrichtung 5 ist zwischen einer Fixierposition 8 und einer Freigabeposition 9 beweglich, wobei der Schlagbolzen 3 in der Fixierposition 8 von dem Schlagstück 6 beabstandet ist. Bei einer axialen Bewegung des Schlagstücks 6 in das Gehäuse 2 hinein bewegt sich die Auslösevorrichtung 5 selbsttätig von der Fixierposition 8 in die Freigabeposition 9.

Nach Erreichen der Freigabeposition 9 ist der Schlagbolzen 3 freigegeben, so dass sich dieser von dem ersten Federelement 4 beschleunigt in Richtung auf das Schlagstück 6 bewegt.

Die Auslösevorrichtung 5 weist eine erste Hülse 10 auf, welche axial beweglich in der Bohrung 22 des Gehäuses 2 angeordnet ist, wobei die erste Hülse 10 den Schlagbolzen 3 aufnimmt, wobei die erste Hülse 10 über den Umfang verteilt drei Durchbrechungen 11 aufweist. In den Durchbrechungen 11 ist jeweils ein Sperrkörper 12 in Form einer Metallkugel angeordnet. Die erste Hülse 10 besteht ebenfalls aus metallischem Werkstoff.

In den Schlagbolzen 3 ist eine Vertiefung 13 in Form einer umlaufenden Nut eingebracht, wobei die Sperrkörper 12 in der Fixierposition 8 in der Vertiefung 13 verrastet sind.

Zwischen der ersten Hülse 10 und dem Gehäuse 2 ist eine zweite Hülse 17 angeordnet, wobei die Innenwand 26 der zweiten Hülse 17 eine Gleitfläche für die Sperrkörper 12 bildet. In der Fixierposition 8 legen sich die Sperrkörper 12 an die Innenwand 26 der zweiten Hülse 17 an. Dabei ist der Innendurchmesser der zweiten Hülse 17 so gewählt, dass die Sperrkörper 12 in den Durchbrechungen 11 der ersten Hülse 10 und in der Vertiefung 13 des Schlagbolzens 3 gehalten sind, so dass der Schlagbolzen 3 relativ zu der ersten Hülse 10 verriegelt ist.

Wird das Einschlag-Instrument 1 auf ein zu fügendes Implantat aufgesetzt und eine Kraft auf das Einschlag-Instrument 1 ausgeübt, verschiebt das Schlagstück 6 die erste Hülse 10 und den mit der ersten Hülse 10 verriegelten Schlagbolzen 3 relativ zu dem Gehäuse 2 in axialer Richtung. Das Schlagstück 6 verschiebt sich dabei in axialer Richtung in das Gehäuse 2 hinein.

Die zweite Hülse 17 ist mit einer Querschnittserweiterung 14 versehen, wobei sich der Innendurchmesser der zweiten Hülse 17 im Bereich der Querschnittserweiterung 14 vergrößert, wobei die Querschnittserweiterung 14 zur teilweisen Aufnahme der Sperrkörper 12 in der Freigabeposition 9 ausgebildet ist.

Wird das Schlagstück 6 so weit axial in das Gehäuse 2 hinein verschoben, erreichen die Sperrkörper 12 in der Freigabeposition 9 den Bereich der Querschnittserweiterung 14. Dabei gleiten die Sperrkörper 12 aus der Vertiefung 13 des Schlagbolzens 3 heraus und in die Durchbrechung 11 der ersten Hülse 10 und die Querschnittserweiterung 14 der zweiten Hülse 17 hinein. Dadurch wird der Schlagbolzen 3 schlagartig freigegeben und durch das erste Federelement 4 in Richtung auf das Schlagstück 6 beschleunigt.

Die erste Hülse 10 weist auf einer Stirnseite eine Aufnahme 15 für das Schlagstück 6 auf, wobei die Aufnahme 15 so ausgebildet ist, dass der Schlagbolzen 3 in der Freigabeposition 9 das Schlagstück 6 unmittelbar kontaktiert. Dazu weist die erste Hülse 10 auf der Stirnseite eine Durchbrechung 25 auf.

Der Schlagbolzen 3 ist mit einem umlaufenden Kragen 16 versehen, wobei sich das erste Federelement 4 an dem Kragen 16 und an dem Gehäuse 2 abstützt.

Die zweite Hülse 17 ist axial verschieblich in dem Gehäuse 2 aufgenommen. Ein zweites Federelement 18 positioniert die zweite Hülse 17 relativ zu dem Gehäuse 2.

Des Weiteren ist ein drittes Federelement 19 vorgesehen, welches stirnseitig zwischen der ersten Hülse 10 und dem Gehäuse 2 angeordnet ist und bewirkt, dass in der Freigabeposition 9 die erste Hülse 10 und das Schlagstück 6 zurückgeführt werden.

Das Gehäuse 2 weist an der dem Schlagstück 6 abgewandten Stirnseite ein Handstück 20 auf. Das Handstück 20 kann aus Kunststoff oder Metall ausgebildet sein.

Zwischen Gehäuse 2 und Schlagstück 6 ist ein Dichtelement 21 vorgesehen.

Das Schlagstück 6 weist auf der dem Schlagbolzen 3 zugewandten Stirnseite einen Vorsprung auf, welcher durch die in die erste Hülse 10 stirnseitig eingebrachte Durchbrechung 25 hindurchragt.

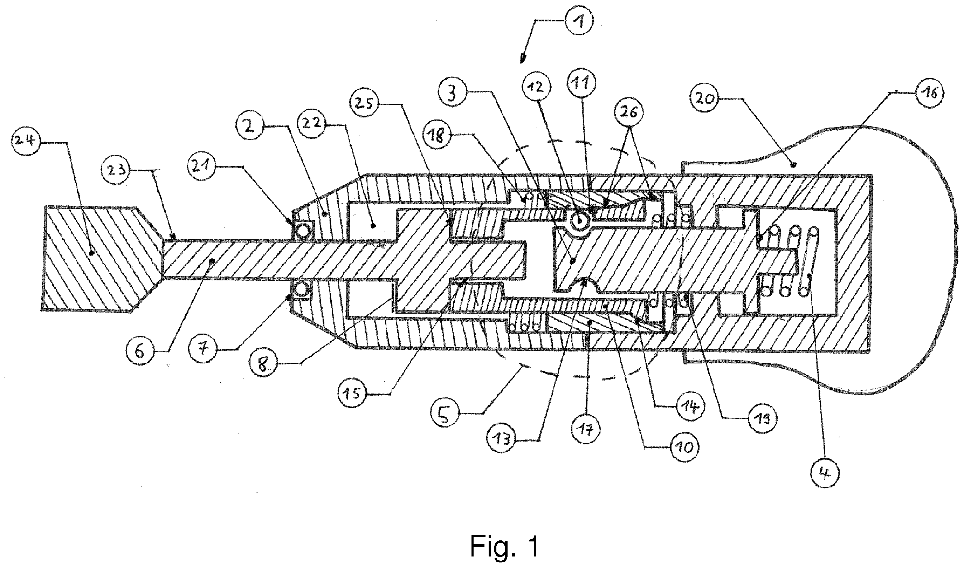

Figur 1 zeigt das Einschlag-Instrument 1 in der Ausgangsstellung. Dabei sind das Schlagstück 6 und der Schlagbolzen 3 voneinander beabstandet. Die Sperrkörper 12 liegen in der Durchbrechung 11 der ersten Hülse 10 und in der Vertiefung 13 des Schlagbolzens 3. Die Sperrkörper 12 stützen sich an der Innenwand 26 der zweiten Hülse 17 ab. Dadurch sind die erste Hülse 10 und der Schlagbolzen 3 miteinander verriegelt.

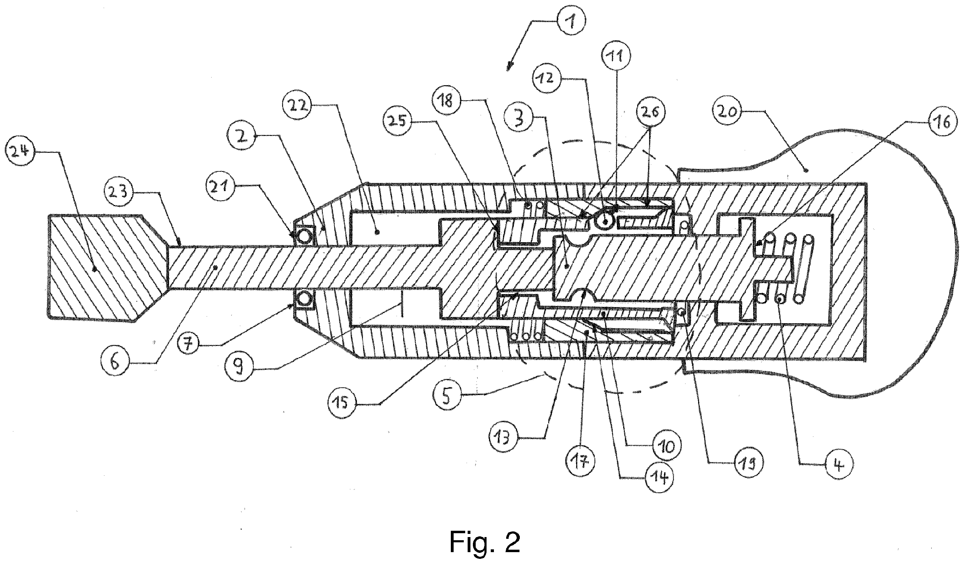

Figur 2 zeigt das Einschlag-Instrument 1, nachdem dieses auf ein zu fügendes Implantatteil aufgesetzt wurde und über das Handstück 20 eine Kraft axial in das Einschlag-Instrument 1 eingebracht wurde. Durch das Einbringen der Kraft in axialer Richtung verschiebt sich das Schlagstück 6 relativ zu dem Gehäuse 2, wobei das Schlagstück 6 die erste Hülse 10 und die darin angeordneten Sperrkörper 12 mitnimmt. Dadurch, dass der Schlagbolzen 3 über die Sperrkörper 12 mit der ersten Hülse 10 verriegelt ist, verschiebt sich auch der Schlagbolzen 3 gleichermaßen.

Bei der in Figur 2 gezeigten Position ist die Querschnittserweiterung 14 der zweiten Hülse 17 erreicht und die Sperrkörper 12 gleiten aus der Vertiefung 13 des Schlagbolzens 3 heraus und geben den Schlagbolzen 3 frei. Durch das Verschieben des Schlagstückes 6, der ersten Hülse 10 und des Schlagbolzens 3 wurde das erste Federelement 4 vorgespannt, so dass der Schlagbolzen 3 nach Freigabe durch die aus der Vertiefung 13 herausgeglittenen Sperrkörper 12 in Richtung auf das Schlagstück 6 beschleunigt wird.

Nach dem Auslösen wird der Schlagbolzens 3 durch das Federelement 4 beschleunigt. Der beschleunigte Schlagbolzen 3 kontaktiert das Schlagstück 6 stirnseitig unmittelbar und mit hoher Geschwindigkeit. Zusammen mit der für das Auslösen der Auslösevorrichtung 5 benötigten Vorkraft wird ein Impuls in das Schlagstück 6 eingebracht, welcher wiederum über das freie Ende 23 in das zu fügende Implantatteil eingebracht wird. Zur besseren Kontaktierung des Implantatteiles weist das Schlagstück 6 an dem freien Ende 23 ein Kontaktierelement 24 auf, welches auf der dem Implantatteil zugewandten Seite mit einer kegelförmigen Vertiefung versehen ist. Damit ist zumindest eine ringförmige Anlage des Schlagstückes 6 an dem zu fügenden Implantatteil gewährleistet. Durch die zum Auslösen der Auslösevorrichtung 5 erforderliche Vorkraft liegt das Einschlag-Instrument 1 beziehungsweise das Kontaktierelement 24 des Schlagstückes 6 mit Vorspannung an dem zu fügenden Implantatteil auf. Dadurch kann der über den Schlagbolzen 3 in das Schlagstück 6 eingebrachte Impuls vollständig auf das zu fügende Implantatteil übertragen werden. Dies ermöglicht das Eindringen eines vorbestimmten und reproduzierbaren Impulses in ein Implantatteil.

Während des Beschleunigens des Schlagbolzens 3 verschiebt sich der Schlagbolzen 3 in axialer Richtung relativ zu der ersten Hülse 10, der zweiten Hülse 17 und dem Schlagstück 6. In dieser Position ist die Vertiefung 13 des Schlagbolzens 3 in axialer Richtung zu den Sperrkörpern 12 verschoben. Die Sperrkörper 12 verharren in der Durchbrechung 11 der ersten Hülse 10 und in der Querschnittserweiterung 14 der zweiten Hülse 17.

Wird nun das Einschlag-Instrument 1 entlastet, bewegt sich das Schlagstück 6 wieder aus dem Gehäuse 2 heraus. Dabei entspannen sich die Federelemente 18, 19, wobei das dritte Federelement 19 bewirkt, dass sich die erste Hülse 10 gemeinsam mit dem Schlagstück 6 in axialer Richtung bewegt und dabei die erste Hülse 10 an der Stirnseite des Schlagstücks 6 anliegt. Das zweite Federelement 18 wiederum bewirkt, dass sich die zweite Hülse 17 so weit in die entgegengesetzte Richtung bewegt, dass die Durchbrechung 11, die Sperrkörper 12 und die Vertiefung 13 fluchten und dadurch die Sperrkörper 12 wieder in die Vertiefung 13 hineingleiten und so den Schlagbolzen 3 mit der ersten Hülse 10 verriegeln.

Somit bewegen sich die Komponenten des Einschlag-Instrumentes 1 nach Ausüben des Schlages und dem Entlasten selbsttätig in die in Figur 1 gezeigte Ausgangsstellung zurück. Das Einschlag-Instrument 1 kann unmittelbar für einen weiteren Schlag eingesetzt werden. Es ist nicht erforderlich, das Gerät manuell vorzuspannen oder dergleichen.

## Patentansprüche

1. Einschlag-Instrument (1) zum Fügen prothetischer Implantate, umfassend ein Gehäuse (2), welches einen Schlagbolzen (3), ein dem Schlagbolzen (3) zugeordnetes erstes Federelement (4) und zumindest teilweise ein Schlagstück (6) aufnimmt, wobei das Schlagstück (6) an einer Stirnseite (7) aus dem Gehäuse (2) hervorsteht, **dadurch gekennzeichnet, dass** das Gehäuse (2) eine Auslösevorrichtung (5) aufnimmt, wobei die Auslösevorrichtung (5) zwischen einer Fixierposition (8) und einer Freigabeposition (9) beweglich ist, wobei der Schlagbolzen (3) in der Fixierposition (8) von dem Schlagstück (6) beabstandet ist, wobei sich die Auslösevorrichtung (5) bei einer axialen Bewegung des Schlagstücks (6) in das Gehäuse (2) hinein selbsttätig von der Fixierposition (8) in die Freigabeposition (9) bewegt, um nach Erreichen der Freigabeposition (9) den Schlagbolzen (3) freizugeben, so dass sich dieser von dem ersten Federelement (4) beschleunigt in Richtung auf das Schlagstück (6) bewegt.

2. Einschlag-Instrument nach Anspruch 1, **dadurch gekennzeichnet, dass** sich das Gehäuse (2) beim Aufsetzen des Einschlag-Instrumentes (1) auf ein zu fügendes Implantat in axialer Richtung in Richtung auf das Implantat bewegt, wobei sich das Schlagstück (6) in das Gehäuse (2) hinein- und von der Fixierposition (8) in die Freigabeposition (9) bewegt, wobei der Schlagbolzen (3) freigeben ist, sobald das Schlagstück (6) die Freigabeposition (9) erreicht und sich der Schlagbolzen (3), beschleunigt durch das erste Federelement (4), in Richtung auf das Schlagstück (6) bewegt, um einen Impuls in das Schlagstück (6) einzubringen.

3. Einschlag-Instrument nach Anspruch 1 oder 2, **dadurch gekennzeichnet, dass** die Auslösevorrichtung (5) eine erste Hülse (10) aufweist, welche axial beweglich in dem Gehäuse (2) angeordnet ist und den Schlagbolzen (3) aufnimmt, wobei die erste Hülse (10) am Umfang zumindest eine Durchbrechung (11) aufweist, wobei in der Durchbrechung (11) zumindest ein Sperrkörper (12) angeordnet ist.

4. Einschlag-Instrument nach Anspruch 3, **dadurch gekennzeichnet, dass** in dem Schlagbolzen (3) zumindest eine Vertiefung (13) eingebracht ist, welche in der Fixierposition (8) den Sperrkörper (12) aufnimmt.

5. Einschlag-Instrument nach Anspruch 4, **dadurch gekennzeichnet, dass** die Vertiefung (13) in Form einer umlaufenden Nut ausgebildet ist, welche in den Umfang des Schlagbolzens (3) eingebracht ist.

6. Einschlag-Instrument nach Anspruch 4 oder 5, **dadurch gekennzeichnet, dass** sich der Sperrkörper (12) in der Fixierposition (8) an die Innenwand (26) des Gehäuses (2) anlegt, wobei der Innendurchmesser des Gehäuses (2) so gewählt ist, dass der Sperrkörper (12) in der Durchbrechung (11) der ersten Hülse (10) und in der Vertiefung (13) des Schlagbolzens (3) gehalten ist und dadurch der Schlagbolzen (3) relativ zu der ersten Hülse (10) verriegelt ist.

7. Einschlag-Instrument nach Anspruch 6, **dadurch gekennzeichnet, dass** das Schlagstück (6) die erste Hülse (10) und den mit der ersten Hülse (10) verriegelten Schlagbolzen (3) in dem Gehäuse (2) axial verschiebt, wenn sich das Schlagstück (6) in axialer Richtung in das Gehäuse (2) hinein verschiebt.

8. Einschlag-Instrument nach einem der Ansprüche 3 bis 6, **dadurch gekennzeichnet, dass** das Gehäuse (2) mit einer Querschnittserweiterung (14) versehen ist, wobei sich der Innendurchmesser des Gehäuses (2) im Bereich der Querschnittserweiterung (14) vergrößert, wobei die Querschnittserweiterung (14) zur teilweisen Aufnahme des Sperrkörpers (12) in der Freigabeposition (9) ausgebildet ist.

9. Einschlag-Instrument nach Anspruch 8, **dadurch gekennzeichnet, dass** der Sperrkörper (12) in der Freigabeposition (9) aus der Vertiefung (13) des Schlagbolzens (3) herausgleitet und in die Durchbrechung (11) der ersten Hülse (10) und die Querschnittserweiterung (14) des Gehäuses (2) hineingleitet, so dass der Schlagbolzen (3) freigegeben ist.

10. Einschlag-Instrument nach einem der Ansprüche 3 bis 9, **dadurch gekennzeichnet, dass** die erste Hülse (10) auf einer Stirnseite eine Aufnahme (15) für das Schlagstück (6) aufweist, wobei die Aufnahme (15) so ausgebildet ist, dass der Schlagbolzen (3) in der Freigabeposition (9) das Schlagstück (6) unmittelbar kontaktiert.

11. Einschlag-Instrument nach einem der Ansprüche 1 bis 10, **dadurch gekennzeichnet, dass** der Schlagbolzen (3) mit einem umlaufenden Kragen (16) versehen ist, wobei sich das erste Federelement (4) an dem Kragen (16) und an dem Gehäuse (2) abstützt.

12. Einschlag-Instrument nach einem der Ansprüche 3 bis 11, **dadurch gekennzeichnet, dass** zwischen der ersten Hülse (10) und dem Gehäuse (2) eine zweite Hülse (17) angeordnet ist, wobei die Innenwand (26) der zweiten Hülse (17) eine Gleitfläche für den Sperrkörper (12) bildet und wobei die Querschnittserweiterung (14) in die zweite Hülse (17) eingebracht ist.

13. Einschlag-Instrument nach Anspruch 12, **dadurch gekennzeichnet, dass** die zweite Hülse (17) axial verschieblich in dem Gehäuse (2) aufgenommen ist und ein zweites Federelement (18) vorgesehen ist, welches die zweite Hülse (17) relativ zu dem Gehäuse (2) positioniert.

14. Einschlag-Instrument nach einem der Ansprüche 3 bis 13, **dadurch gekennzeichnet, dass** ein drittes Federelement (19) vorgesehen ist, welches stirnseitig zwischen der ersten Hülse (10) und dem Gehäuse (2) angeordnet ist.

15. Einschlag-Instrument nach einem der Ansprüche 1 bis 14, **dadurch gekennzeichnet, dass** das Gehäuse (2) mit einem Handstück (20) versehen ist.

## Claims

1. Tap-in instrument (1) for joining prosthetic implants, comprising a housing (2), which receives a firing pin (3), a first spring element (4) assigned to the firing pin (3) and, at least in part, a striker (6), the striker (6) protruding from the housing (2) at an end face (7), **characterised in that** the housing (2) receives a trigger device (5), the trigger device (5) being movable between a fixing position (8) and a release position (9), the firing pin (3) being spaced apart from the striker (6) in the fixing position (8), the trigger device (5) moving from the fixing position (8) into the release position (9) automatically upon an axial movement of the striker (6) into the housing (2), so as to release the firing pin (3) after reaching the release position (9), in such a way that said pin moves towards the striker (6), accelerated by the first spring element (4).

2. Tap-in instrument according to claim 1, **characterised in that** when the tap-in instrument (1) is placed in an axial direction on an implant to be joined the housing (2) moves towards the implant, the striker (6) moving into the housing (2) and from the fixing position (8) into the release position (9), the firing pin (3) being released once the striker (6) reaches the release position (9), and the firing pin (3), accelerated by the first spring element (4), moving towards the striker (6) to introduce an impulse into the striker (6).

3. Tap-in instrument according to either claim 1 or claim 2, **characterised in that** the trigger device (5) has a first sleeve (10), which is arranged axially movably in the housing (2) and receives the striker pin (3), the first sleeve (10) having on the circumference at least one clearance (11), at least one blocking body (12) being arranged in the clearance (11).

4. Tap-in instrument according to claim 3, **characterised in that** at least one depression (13) is formed in the firing pin (3) and receives the blocking body (12) in the fixing position (8).

5. Tap-in instrument according to claim 4, **characterised in that** the depression (13) is in the form of a circumferential groove formed in the circumference of the firing pin (3).

6. Tap-in instrument according to either claim 4 or claim 5, **characterised in that** the blocking body (12) is positioned against the inner wall (26) of the housing (2) in the fixing position (8), the internal diameter of the housing (2) being selected in such a way that the blocking body (12) is held in the clearance (11) of the first sleeve (10) and in the depression (13) of the firing pin (3) and the firing pin (3) is thus locked relative to the first sleeve (10).

7. Tap-in instrument according to claim 6, **characterised in that** the striker (6) axially displaces the first sleeve (10) and the firing pin (3) locked to the first sleeve (10) in the housing (2) when the firing pin (6) is displaced into the housing (2) in an axial direction.

8. Tap-in instrument according to any of claims 3 to 6, **characterised in that** the housing (2) is provided with a cross-sectional extension (14), the internal diameter of the housing (2) increasing in the region of the cross-sectional extension (14), the cross-sectional extension (14) being formed to receive the blocking body (12) in part in the release position (9).

9. Tap-in instrument according to claim 8, **characterised in that** in the release position (9) the blocking body (12) slides out of the depression (13) in the firing pin (3), into the clearance (11) in the first sleeve (10) and into the cross-sectional extension (14) of the housing (2), in such a way that the firing pin (3) is released.

10. Tap-in instrument according to any of claims 3 to 9, **characterised in that** the first sleeve (10) has a recess (15) for the striker (6) on an end face, the recess (15) being formed in such a way that the firing pin (3) directly contacts the striker (6) in the release position (9).

11. Tap-in instrument according to any of claims 1 to 10, **characterised in that** the firing pin (3) is provided with a circumferential collar (16), the first spring element (4) being braced on the collar (16) and on the housing (2).

12. Tap-in instrument according to any of claims 3 to 11, **characterised in that** a second sleeve (17) is arranged between the first sleeve (10) and the housing (2), the inner wall (26) of the second sleeve (17) forming a sliding face for the blocking body (12), and the cross-sectional extension (14) being formed in the second sleeve (17).

13. Tap-in instrument according to claim 12, **characterised in that** the second sleeve (17) is received axially displaceably in the housing (2), and a second spring element (18) is provided, which positions the second sleeve (17) relative to the housing (2).

14. Tap-in instrument according to any of claims 3 to 13, **characterised in that** a third spring element (19) is provided, which is arranged at the end face between the first sleeve (10) and the housing (2).

15. Tap-in instrument according to any of claims 1 to 14, **characterised in that** the housing (2) is provided with a handpiece (20).

## Revendications

1. Instrument à percussion (1) pour assembler des implants prothétiques, comprenant un boîtier (2) qui reçoit un percuteur (3), un premier élément à ressort (4) associé au percuteur (3) et, au moins en partie, une pièce de percussion (6), dans lequel la pièce de percussion (6) fait saillie à partir du boîtier (2) au niveau d'une face frontale (7), **caractérisé en ce que** le boîtier (2) reçoit un dispositif de déclenchement (5), dans lequel le dispositif de déclenchement (5) est mobile entre une position de fixation (8) et une position de libération (9), dans lequel, dans la position de fixation (8), le percuteur (3) est espacé de la pièce de percussion (6), dans lequel, lorsque la pièce de percussion (6) se déplace axialement dans le boîtier (2), le dispositif de déclenchement (5) se déplace automatiquement de la position de fixation (8) à la position de libération (9) afin de libérer le percuteur (3) une fois que la position de libération (9) est atteinte, de telle sorte que ce dernier, accéléré par le premier élément à ressort (4), se déplace en direction de la pièce de percussion (6).

2. Instrument à percussion selon la revendication 1, **caractérisé en ce que** lorsque l'instrument à percussion (1) est placé sur un implant à assembler, le boîtier (2) se déplace dans une direction axiale vers l'implant, dans lequel la pièce de percussion (6) se déplace dans le boîtier (2) et de la position de fixation (8) à la position de libération (9), dans lequel le percuteur (3) est libéré dès que la pièce de percussion (6) atteint la position de libération (9) et le percuteur (3), accéléré par le premier élément à ressort (4), se déplace en direction de la pièce de percussion (6), afin d'introduire une impulsion dans la pièce de percussion (6).

3. Instrument à percussion selon la revendication 1 ou 2, **caractérisé en ce que** le dispositif de déclenchement (5) comporte une première douille (10) qui est agencée dans le boîtier (2) de manière axialement mobile et qui reçoit le percuteur (3), dans lequel la circonférence de la première douille (10) comporte au moins une ouverture (11), dans lequel au moins un corps de blocage (12) est agencé dans l'ouverture (11).

4. Instrument à percussion selon la revendication 3, **caractérisé en ce qu'**au moins un évidement (13) est inséré dans le percuteur (3), lequel évidement reçoit le corps de blocage (12) dans la position de fixation (8).

5. Instrument à percussion selon la revendication 4, **caractérisé en ce que** l'évidement (13) est conçu sous la forme d'une gorge circonférentielle qui est réalisée dans la circonférence du percuteur (3).

6. Instrument à percussion selon la revendication 4 ou 5, **caractérisé en ce que**, dans la position de fixation (8), le corps de blocage (12) vient s'appliquer contre la paroi intérieure (26) du boîtier (2), dans lequel le diamètre intérieur du boîtier (2) est sélectionné de telle sorte que le corps de blocage (12) est maintenu dans l'ouverture (11) de la première douille (10) et dans l'évidement (13) du percuteur (3), et le percuteur (3) est ainsi bloqué par rapport à la première douille (10).

7. Instrument à percussion selon la revendication 6, **caractérisé en ce que** la pièce de percussion (6) déplace axialement la première douille (10) et le percuteur (3) bloqué avec la première douille (10) dans le boîtier (2), lorsque la pièce de percussion (6) se déplace dans une direction axiale dans le boîtier (2).

8. Instrument à percussion selon l'une des revendications 3 à 6, **caractérisé en ce que** le boîtier (2) est muni d'un élargissement de section transversale (14), dans lequel le diamètre intérieur du boîtier (2) augmente dans la zone de l'élargissement de section transversale (14), dans lequel l'élargissement de section transversale (14) est conçu pour recevoir partiellement le corps de blocage (12) dans la position de libération (9).

9. Instrument à percussion selon la revendication 8, **caractérisé en ce que**, dans la position de libération (9), le corps de blocage (12) glisse en dehors de l'évidement (13) du percuteur (3) et glisse dans l'ouverture (11) de la première douille (10) et l'élargissement de section transversale (14) du boîtier (2), de telle sorte que le percuteur (3) est libéré.

10. Instrument à percussion selon l'une des revendications 3 à 9, **caractérisé en ce que** la première douille (10) comporte, au niveau d'un côté avant, une partie de réception (15) pour la pièce de percussion (6), dans lequel la partie de réception (15) est conçue de telle sorte que le percuteur (3) dans la position de libération (9) est directement en contact avec la pièce de percussion (6).

11. Instrument à percussion selon l'une des revendications 1 à 10, **caractérisé en ce que** le percuteur (3) est muni d'une collerette circonférentielle (16), dans lequel le premier élément à ressort (4) est en appui contre la collerette (16) et contre le boîtier (2).

12. Instrument à percussion selon l'une des revendications 3 à 11, **caractérisé en ce qu'**entre la première douille (10) et le boîtier (2) est agencée une seconde douille (17), dans lequel la paroi intérieure (26) de la seconde douille (17) forme une surface de glissement pour le corps de blocage (12) et dans lequel l'élargissement de section transversale (14) est introduit dans la seconde douille (17).

13. Instrument à percussion selon la revendication 12, **caractérisé en ce que** la seconde douille (17) est reçue dans le boîtier (2) de manière axialement mobile et un deuxième élément à ressort (18) est prévu, lequel positionne la seconde douille (17) par rapport au boîtier (2).

14. Instrument à percussion selon l'une des revendications 3 à 13, **caractérisé en ce qu'**un troisième élément à ressort (19) est prévu, lequel est agencé du côté avant entre la première douille (10) et le boîtier (2).

15. Instrument à percussion selon l'une des revendications 1 à 14, **caractérisé en ce que** le boîtier (2) est muni d'une pièce à main (20).
